# EUROPEAN PATENT SPECIFICATION

(11) **EP 2 304 028 B1**
(45) Date of publication and mention of the grant of the patent: **25.10.2017**
(21) Application number: 09751727.0
(22) Date of filing: 22.05.2009
(51) Int. Cl.: C12N 15/10

(54) **METHODS AND KITS FOR EXTRACTION OF DNA**
METHODEN UND MATERIAL ZUR EXTRAKTION VON DNA
PROCÉDÉS ET KITS POUR L'EXTRACTION D'ADN

(30) Priority: 23.05.2008 US 55845 P
(43) Date of publication of application: 06.04.2011
(60) Divisional application: 13169345.9
(73) Proprietor: Life Technologies Corporation, Carlsbad, CA 92008 (US)
(72) Inventor: BREVNOV, Maxim, G., Union City, CA 94587 (US); PAWAR, Hemant, S., Ann Arbor, Michigan 48105 (US); FURTADO, Manohar, San Ramon, CA 94583 (US); SHEWALE, Jaiprakash, G., Santa Clara, CA 95051 (US)
(74) Representative: Grund, Martin
(86) International application number: PCT/US2009/045092
(87) International publication number: WO 2009/143499

(56) References cited:
- WO-A-97/08547
- WO-A-2005/089929
- BREVNOV MAXIM G ET AL: "Developmental Validation of the PrepFiler (TM) Forensic DNA Extraction Kit for Extraction of Genomic DNA from Biological Samples", JOURNAL OF FORENSIC SCIENCES, CALLAGHAN AND CO, CHICAGO, IL, US, vol. 54, no. 3, 1 May 2009 (2009-05-01), pages 599-607, XP002541660, ISSN: 0022-1198, DOI: 10.1111/J.1556-4029.2009.01013.X

## Description

### INTRODUCTION

The present teachings provide a composition, methods and kits for obtaining nucleic acids in high quantity, high quality and accurately within a short time. The present teachings are generally directed to methods of isolating nucleic acids from biological materials, such that the nucleic acids are compatible with use in downstream applications. In various embodiments, the present teachings relate to the use of polyhydroxy polymers and detergents for binding of nucleic acids to, release of nucleic acids from, magnetic particles with hydrophilic surfaces, and extracting nucleic acids by using several buffers. In some embodiments, kits are provided for isolating DNA from biological materials.

The extraction and purification methods of the present teachings provide useful methods for obtaining nucleic acids such as genomic DNA from a biological sample, a food sample, a water sample, an environmental sample, an agricultural sample, a biopharmaceutical sample, or a pharmaceutical sample, which can be used in downstream applications such as genotyping, detection, quantification, and identification of the source of the biological, food, water, environmental, agricultural, biopharmaceutical or pharmaceutical material, wherein molecular biological processes such as PCR are utilized. The buffer system provided is unique in extracting high quantity DNA which preserves the DNA integrity. It provides high efficiency of DNA extraction as well as the removal of PCR inhibitors. Furthermore, the procedure for extraction and purification of nucleic acids is fully automatable, using standard liquid handling systems.

The section headings used herein are for organizational purposes only and are not to be construed as limiting the subject matter described in any way.

### DRAWINGS

The skilled artisan will understand that the drawings, described below, are for illustration purposes only. The drawings are not intended to limit the scope of the present teachings in any way.
FIG. 1 is a schematic demonstrating a method of DNA isolation and purification, as described in various embodiments of the present teachings.
FIG. 2 demonstrates DNA yield from DNA isolation and purification as described in Example 6, wherein genomic DNA was isolated from cultured Raji cells at cell counts varying from 1562 to 50000, according to the methods described in Example 4.
FIG. 3 demonstrates DNA yield from DNA isolation and purification as described in Example 7, wherein genomic DNA was isolated from cultured K562 cells at cell counts varying from 3500 to 110000, according to the methods as described in the Example 4.
FIG. 4 demonstrates genotyping profiles obtained following DNA isolation and purification from substrates as described in Example 13, wherein genomic DNA was isolated from biological samples according to the methods of Example ] 1, and was processed for genotyping using the Identifiler® kit.
Figure 5. Influence of detergents additives to wash solution on DNA extraction from blood dried on denim in presence of inhibitors.
Figure 6. Influence of detergents additives to wash solution on efficiency to remove inhibitors from blood dried on denim in presence of inhibitors.

### DESCRIPTION OF VARIOUS EMBODIMENTS

While the present teachings are described in conjunction with various embodiments, it is not intended that the present teachings be limited to such embodiments. The invention is defined by the appended claims.

Most of the words used in this specification have the meaning that would be attributed to those words by one skilled in the art. Words specifically defined in the specification have the meaning provided in the context of the present teachings as a whole, and as are typically understood by those skilled in the art. In the event that a conflict arises between an art-understood definition of a word or phrase and a definition of the word or phrase as specifically taught in this specification, the specification shall control. Headings used herein are merely for convenience, and are not to be construed as limiting in any way.

As used herein, "DNA" refers to deoxyribonucleic acid in its various forms as understood in the art, such as genomic DNA, cDNA, isolated nucleic acid molecules, vector DNA, and chromosomal DNA. "Nucleic acid" refers to the nucleic acid molecule or molecules, DNA or RNA (ribonucleic acid) in any form. As used herein, the term "isolated nucleic acid molecule" or "isolated nucleic acid" refers to a nucleic acid molecule (DNA or RNA of any form) that has been removed from its native environment. Some examples of isolated nucleic acid molecules are recombinant DNA molecules contained in a vector, recombinant DNA molecules maintained in a heterologous host cell, partially or substantially purified nucleic acid molecules, nucleic acids obtained from forensic and other samples comprising biological material, such as blood, semen, saliva, skin tissue, etc., food samples including but not limited too, meat, fish, fruit, vegetables, beer, wine, eggs, milk , etc., and samples of plant, animal, human or environmental origin, a water sample, an environmental sample, an agricultural sample, a biopharmaceutical sample, a pharmaceutical sample, environmental samples present in raw materials, equipment, products or processes used to manufacture or store food, beverages, water, pharmaceutical products, personal care products or dairy products, in clinical specimens, equipment, fixtures or products used to treat humans or animals as well as in clinical samples and clinical environments, and synthetic DNA molecules. An "isolated" nucleic acid can be free of sequences which naturally flank the nucleic acid (i.e., sequences located at the 5' and 3' ends of the nucleic acid) in the genomic DNA of the organism from which the nucleic acid is derived. Moreover, an "isolated" nucleic acid molecule, such as a cDNA molecule, can be substantially free of other cellular material or culture medium when produced by recombinant techniques, or of chemical precursors or other chemicals when chemically synthesized.

"Polymerase chain reaction" (or "PCR") refers to a technique in which repetitive cycles of denaturation, annealing with a primer, and extension with a DNA polymerase enzyme are used to amplify the number of copies of a target DNA sequence by approximately 10⁶ times or more. The PCR process for amplifying nucleic acids is covered by U.S. Patent Nos. 4,683,195 and 4,683,202. The reaction conditions for any PCR comprise the chemical components of the reaction and their concentrations, the temperatures used in the reaction cycles, the number of cycles of the reaction, and the durations of the stages of the reaction cycles.

"PCR-compatible" refers to any composition, solution, compound, reagent, etc. that is compatible with subsequent use in PCR assays and is relatively non- inhibitory of the enzymatic polymerase chain reaction. PCR-compatible products demonstrate relatively minimal or no inhibition of PCR amplification, as evidenced by comparison of PCR results with the relevant positive and negative controls. PCR assays can include, but are not limited to, DNA genotyping systems, TaqMan® or SYBR® green real-time PCR assays for DNA quantification, multiplex PCR assays including those designed to genotype short tandem repeats, etc.

As used herein, "amplify" refers to the process of enzymatically increasing the amount of a specific nucleotide sequence. This amplification is not limited to but is generally accomplished by PCR.

"Polymer" or "polymers" refer to soluble polyhydroxy polymers for binding of nucleic acids to, and release of nucleic acids from, magnetic particles with hydrophilic surfaces.

In some embodiments of the present teachings, methods are described in which nucleic acid molecules can be separated and/or isolated from samples and, in some embodiments, in which the product made from the methods are nucleic acids. In some embodiments, the methods of the present teachings result in the formation of a product which comprises the isolated nucleic acid.

In some embodiments of the present teachings, nucleic acid molecules can be separated and/or isolated from samples containing biological materials and, in some embodiments, the product made from the methods are nucleic acids. Examples of such samples include but are not limited to blood and blood stains, saliva and saliva stains, buccal cells and buccal swabs, semen and semen stains, cigarette butts, chewing gum, ground beef, brie cheese, raw chicken, shrimp, cantaloupe, dry infant formula, whole shell eggs, ground black pepper, dry pet food, peanut butter, orange juice, pasteurized milk, alfalfa sprouts, roast beef, smoked salmon, mayonnaise, salad dressing, milk, ice cream, cured bacon, lettuce, sausages, beet trim, juices, spinach, cheddar cheese, raw milk, oysters, clams, and mussels.

In the present teachings, methods are described wherein cells from a sample are lysed in lysis solution, forming a lysate comprising nucleic acid. The lysate is treated with a starting solution comprising water-soluble polyhydroxy polymers and detergent, and magnetically attractable dextran-encased magnetite particles are added in order to recover nucleic acid molecules from the sample by applying a magnetic field. In various embodiments, the sample can comprise one or more of cells; biological materials such as buccal swabs, stained fabrics, and so on. The methods further comprise the step of releasing the nucleic acid from the magnetically attractable particles by eluting the nucleic acid in an aqueous solution.

The nucleic acids thus obtained can then be utilized in any of various downstream applications such as, for example, quantification, detection, and genotyping of specific nucleic acids or even of a biological species. These analyses can be performed, for example, by PCR amplification. As one example, in forensic DNA analysis the human nuclear DNA (nDNA) and/or genomic DNA can be obtained from complex biomaterials and then genotyped using PCR. As another example, a DNA preparation can be used for quantification of human DNA, or more specifically human male DNA, using real-time PCR systems such as Quantifier®, and/or genotyped for autosomal or Y-chromosomal short tandem repeat loci using systems such as, for example, AmpF/STR® kits. Based upon the amount of DNA present in a sample, a particular genotyping system can be selected that will yield the best results for the particular analysis required. Therefore, in order to best utilize nucleic acids in downstream applications, it is particularly desirable that the extraction and isolation methods result not only in a product of high yield, but also one that is relatively free of inhibitors of downstream applications such as PCR.

As an example, typical forensic evidence samples are often exposed to various environmental insults during acquisition and processing, which can lead to contamination with compounds that act to inhibit PCR, and which therefore interfere with attempts at genotyping or other analyses. It is desirable to remove such inhibitors during the isolation of DNA and prior to amplification.

Another example of the use of the nucleic acids is in the detection of contaminating DNA in the quality control of food processing and biopharamaceutical manufacturing.

Typically a portion of food or beverage is combined with an appropriate liquid, including without limitation water, a buffer solution, or a culture medium, including without limitation, a selective medium or an enrichment medium. In some embodiments, the food is chopped, macerated, liquefied, diced, or homogenized. In some embodiments, large volumes of sample, for example but not limited to, volumes of 100 mL, 250 mL, or more are processed according to the disclosed methods to determine if foreign nucleic acid(s) is present in the starting material. According to certain embodiments, a portion of the food or beverage and appropriate liquid are typically combined to form a dilute suspension, for example but not limited to, ratios of about 1:5, 1:10, or 1:20 (w/vol). In some embodiments, a detergent, an emulsifying agent, or both, is added to enhance the solubility of high lipid foods, for example but not limited to butter and certain other dairy products. Those in the art will appreciate that the choice of liquid used to suspend the food or beverage will depend, at least in part, on the starting material (i.e., the food or beverage) and the foreign nucleic acid, including but not limited to, microorganism(s) of interest; and that the food/beverage to liquid ratio can vary widely, provided that the suspension is sufficiently fluid to process, for example but not limited to, passing it through a filtration media. In certain embodiments, 25 grams of a solid or semi-solid food is combined with 225 mL of a suitable culture media. In some embodiments, 25 mL of a beverage or a liquefied or partially liquefied food is combined with 225 mL of a suitable culture media.

The present teachings also relate to efficient binding of nucleic acids such as, for example, genomic DNA to magnetic particles (i.e., magnetically attractable particles, or beads) in such a form that the bound nucleic acids can then be released under the appropriate aqueous conditions. Embodiments of the teachings thus enable effective isolation of nucleic acids, such as genomic DNA, from various different types of biological materials. In addition, nucleic acids such as genomic DNA can be isolated from either small or large quantities of the biological materials that are commonly processed in laboratories such as, for example, those involved in genotyping analyses.

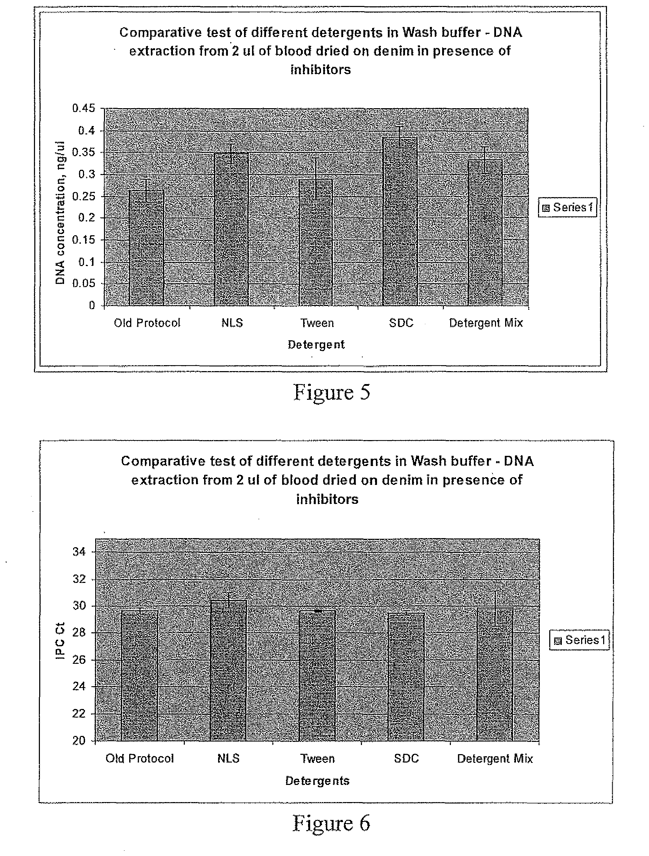

As used herein, wash buffer or wash solution comprises a mixture of anionic detergents (preferably ranging from 0.1 % to 2%), such as N-lauroyl sarcosine, sodium deoxycholate, CTAB, N-dodecyl β -D-rnaltoside, nonanoyl-N-methylglucamide, Triton® X-100 and/or sodium dodecyl sulfate; and a polar solvent such as isopropanol or ethanol (preferably ranging from 50% to 90%). Optionally, the wash solution can also comprise Tween 20, deoxycholate and lauroyl sarcosinate, also known as sarcosyl. Figures 5 and 6 provide comparisons of various detergents in wash buffer. The wash buffer washes the beads after DNA binding and also removes the DNA inhibitors.

These embodiments and other features of the present teachings will become more apparent from the description herein.

### Nucleic acid isolation system

Various embodiments of the present teachings relate to a system, amenable to assembly in a kit, for the binding of nucleic acids such as genomic DNA to magnetic particles having a hydrophilic surface via water-soluble polyhydroxy polymers, in the presence of detergents, forming a nucleic acid-polymer-particle complex, and the production and isolation of such a complex, wherein the nucleic acid does not directly bind to the magnetic particles. The formation of the complex is such that the polymer entraps the nucleic acid, polymer attaches to the particles, and so indirectly connects the nucleic acid with the particles. Various embodiments comprise a lysis solution, which causes the lysis of cells and release of nucleic acid, while protecting the nucleic acid from degradation and removing PCR inhibitors. In the present teachings the nucleic acids remain bound to the magnetic particles via the complex in the presence of a wash solution, in which the complex is washed so as to remove contaminants and inhibitors and so that the nucleic acid is amendable to use in downstream applications, such as PCR. Solutions for washing nucleic acid isolations of any contaminants and inhibitors are well-known to those of skill in the art, and can comprise, for example, detergents and polar solvents. In embodiments of the present teachings, the nucleic acids can then be released in an aqueous solution, such as a buffer, which is also compatible for use in downstream applications such as PCR. A plurality of washes can be performed in the methods of the present invention, separately or in conjunction with a plurality of applications of the magnetic field to the sample to recover and/or separate the nucleic acids.

Standard nucleic acid extraction techniques, including cell lysis, and washing and elution of nucleic acids, are well known in the art and unless otherwise noted, can be carried out according to various techniques as described, for example, in DNA Typing Protocols: Molecular Biology and Forensic Analysis, 1st edition, B. Budowle et al., eds., Eaton Publishing Co. (2000); JM Butler, Forensic DNA Typing: Biology, Technology, and Genetics of STR Markers, 2nd edition, Elsevier Academic Press (2005); or P. Gill, "Application of Low Copy Number DNA Profiling," Croatian Medical Journal42:229-232 (2001); FR Bieber et al., "Isolation of DNA from Forensic Evidence," Current Protocols in Human Genetics, Supplement 26 (2000); Forensic DNA Profiling Protocols, Methods in Molecular Biology, vol. 98, PJ Lincoln and J. Thomson, eds., Humana Press (1998).

Various embodiments of the present teachings relate to a nucleic acid isolation system, such as for genomic DNA, comprising reagents and methods for extraction of the nucleic acids from biological samples, a food sample, a water sample, an environmental sample, an agricultural sample, a biopharmaceutical sample, or a pharmaceutical sample. Embodiments of these methods comprise: lysis of cells from biological material forming a non-covalent complex of nucleic acid (e.g., genomic DNA) with water-soluble polyhydroxy polymers having the same or similar chemical structure as the surface of magnetically attractable particles, in the presence of detergents; binding of the nucleic acid-polymer complex to dextran-encased magnetic particles via interactions between the polymers and surfaces of the particles, thus forming a stable nucleic acid-polymer-particle complex; removal of unbound materials such as PCR inhibitors, via a washing buffer comprising detergent and polar solvent; and releasing the nucleic acid from the particles, and eluting the nucleic acid in an aqueous solution.

Applicants have found that the use of polyhydroxy water-soluble polymers and detergent, in the presence of appropriate salts and polar solvent, improves the efficiency of the binding and release of nucleic acids such as genomic DNA on the surface of magnetically attractable particles. An example of appropriate magnetically attractable particles is, but is not limited to, dextran-encased Nanomag® magnetite nanoparticles. In some embodiments of the present teachings, dextran-encased magnetite nanoparticles are added to a sample comprising nucleic acids in the range of about 2 to about 10mg/ml.

The soluble polymers and detergent may be termed binding enhancers. Some examples of appropriate polyhydroxy water-soluble polymers are, but are not limited to, long-chain branched polysaccharides such as dextrans (e.g., dextran 5,000,000 - 40,000,000), soluble starch, dextrins, cellodextrins, polyethylene glycol (PEG), heparin, glycogen, short-chain cellulose, cellulose derivatives, and combinations thereof. Some examples of appropriate detergents are, but are not limited to, N-lauroyl sarcosine (NLS); lauroyl sarcosinate, also known as sarcosyl, an ionic surfactant derived from sarcosine; hexadecyltrimethylammonium bromide or cetyltrimethyiammonium bromide (CTAB); deoxycholate; dodecyl β-D-maltoside; nonanoyl-N-methylglucamide; sodium dodecyl sulfate; polyethylene glycol p-(1,1,3,3-tetramethylbutyl)-phenyl ether (commercially known as Triton® X-100); and combinations thereof. In some embodiments, the binding enhancer comprises dextran in the range of 1-5mg/ml and sarcosyl in the range of about 5 to about 15%.

Some examples of appropriate polar solvents are, but are not limited to, isopropanol, ethanol, butanol, and combinations thereof. In some embodiments, the polar solvent comprises about 70% to about 100% isopropanol.

In some embodiments, magnetically attractable particles can be added to a sample comprising nucleic acid, such as a cell lysate, at the same time as the binding enhancers, forming a suspension. A solution comprising polar solvent can then be added to this suspension. Alternatively, in some embodiments of the present teachings a single solution comprising binding enhancers and polar solvent can be added to the suspension. Binding enhancers, solvent and cell lysate provide unique conditions such that nucleic acids are entrapped in a non-covalent complex with soluble polymers having the same or a similar chemical structure as the surface of the magnetic particles. The result is the effective binding of the polymer-entrapped nucleic acids to the magnetic particles in a non-covalent nucleic acid-polymer-particle complex. This complex is stable under alcohol wash conditions, and the nucleic acids can easily be later eluted in a standard low-salt buffer such as, for example, Tris buffer containing low concentrations of divalent metal ion chelating agents, such as EDTA. In some embodiments, this stage of nucleic acid-polymer binding to particles may be assisted by chilling.

Additionally, the present teaching provides that using detergents (such as, but not limited to, N-lauroyl sarcosine, sodium deoxycholate, CTAB, N-dodecyl β -D-maltoside, nonanoyl-N-methylglucamide, Triton® X-100 and/or sodium dodecyl sulfate) mixed with a polar solvent (such as, for example, ethanol or isopropanol) as a wash solution of nucleic acid-particle complexes to improve the removal of inhibitors of downstream applications, such as PCR inhibitors, from these nucleic acids bound to the magnetic particles.

Following binding of the polymer-entrapped nucleic acids to magnetic particles in the formation of the nucleic acid-polymer-particle complexes, a magnetic field is then applied to the suspension. This magnetic field is used to remove the nucleic acid-polymer-particle complexes from the suspension, forming a complex layer, for example, at the bottom or side of the tube and leaving a supernatant. The magnetic field can be applied to the sample by devices and methods known in the art (e.g., via Ambion® (Austin, TX) Magnetic Stands). The supernatant can then be removed from the tube.

The nucleic acid-polymer-particle complex layer is then washed, to remove residual contaminants and/or inhibitors of PCR; then the nucleic-acid- polymer-particle complex can be resuspended in the requisite volume of an appropriate elution buffer in the absence of any magnetic field. The appropriate elution buffers for the isolation of nucleic acids are well-known to those of skill in the art. These allow for release of the nucleic acids into solution from the nucleic acid-polymer-particle complexes. A magnetic field can be reapplied to the tube, resulting in removal of the magnetic particles from the suspension to, for example, the bottom or side of the tube, leaving a supernatant in which the nucleic acid is now dissolved. The redissolved nucleic acid can now be separated from the magnetic particles by collecting the supernatant with, for example, a pipette while the particles are held against the bottom or side of the tube by the magnetic field. Centrifugation of the sample is not required in. these methods.

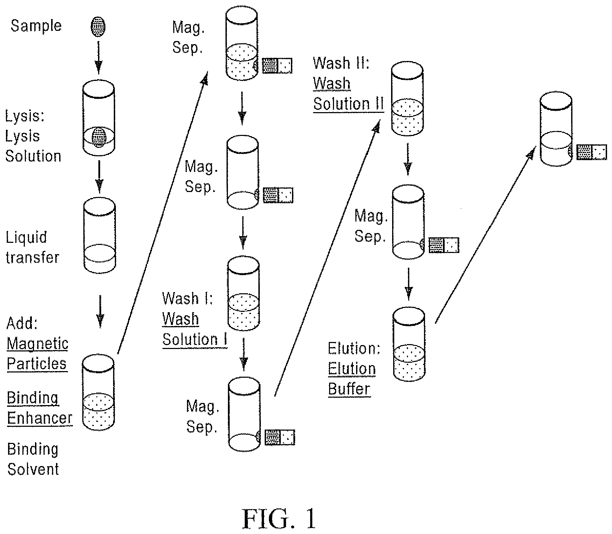

In some embodiments of the present teachings, then, nucleic acid molecules can be isolated from biological, food, water, environmental, agricultural, biopharmaceutical or pharmaceutical samples containing biological materials and purified from solution in combination with the use of magnetic dextran particles. Magnetic particle-facilitated nucleic acid isolation and purification can be used to greatly improve upon the traditional process of alcohol-based precipitation isolation and purification of nucleic acids, well-known to those of skill in the art. An example of an embodiment of an alcohol-based isolation and purification procedure as modified by these teachings can be demonstrated by reference to Fig. 1.

### Sample Preparation

Embodiments of the methods described here can comprise combining a portion of food or beverage with an appropriate liquid, including without limitation water, a buffer solution, or a culture medium, including without limitation, a selective medium or an enrichment medium. In some embodiments, the food is chopped, macerated, liquefied, diced, or homogenized. In some embodiments, large volumes of sample, for example but not limited to, volumes of 100 mL, 250 mL, or more are processed according to the disclosed methods to determine whether a particular microorganism is present in the starting material. According to certain embodiments, a portion of the food or beverage and appropriate liquid are typically combined to form a dilute suspension, for example but not limited to, ratios of about 1:5, 1:10, or 1:20 (w/vol). In some embodiments, a detergent, an emulsifying agent, or both, is added to enhance the solubility of high lipid foods, for example but not limited to butter and certain other dairy products. Those in the art will appreciate that the choice of liquid used to suspend the food or beverage will depend, at least in part, on the starting material (i.e., the food or beverage) and the microorganism (s) of interest; and that the food/beverage to liquid ratio can vary widely, provided that the suspension is sufficiently fluid to process, for example but not limited to, passing it through a filtration media. In certain embodiments, 25 grams of a solid or semi-solid food is combined with 225 mL of a suitable culture media. In some embodiments, 25 mL of a beverage or a liquefied or partially liquefied food is combined with 225 mL of a suitable culture media.

### Lysis solution

Embodiments of the methods described herein comprise the lysing of cells from biological materials present on a substrate such as, for example, the cotton of a buccal swab or a stained fabric, to create a lysate comprising nucleic acids; removing the substrate from the lysate; forming the nucleic acid-polymer-particle complex, followed by separating and eluting of nucleic acids as described above. In one embodiment, the lysis solution can comprise SDS, Tris buffer and NaCl, optionally with proteinase K; and in some embodiments the lysis solution can comprise 0.0%-1% SDS, 10OmM Tris buffer, and 2 M NaCl.

In some embodiments of the present teachings, the lysis solution can comprise guanidinium thiocyanate (GuSCN) or guanidium HCl, TrisHCl, ethylene diamine tetraacetate (EDTA), Antifoam A and an amphiphilic detergent such as a dimethylammonio-propane-1-sulfonate (for example a Zwittergent®, e.g. Zwittergent® 3-16, having a C16 chain). In some embodiments of the present teachings the lysis solution comprises GuSCN in the range of 3.5-6M; EDTA in the range of 10-150mM; TrisHCl in the range of 100-500mM; Antifoam A in the range of 0.005-0.05%; Zwittergent® in the range of 1-5%; with a pH in the range of 7.2-8.5. In some embodiments of the present teachings the lysis solution can further comprise a strong reducing agent such as, for example, tris(2-carboxyethyl)phosphine (TCEP) or dithiothreitol (DTT). Because a strong reducing agent acts to hydrolyze and break protein disulfide bonds, these are especially useful, for example, where the biological material is sperm, because the agents can act to hydrolyze proteins which keep the sperm wall intact, and which therefore make lysis of the sperm cell relatively difficult.

In the present teachings, a lysis solution is added to a sample containing biological material (and optionally, the sample subjected to heat for some time, e.g., twenty minutes to two hours) in order to lyse cells and free nucleic acids into a lysate. In some embodiments the lysis solution can be a composition comprising compounds designed to effectively lyse cells, e.g., buccal cells collected on a cotton swab, while also protecting released nucleic acids from degradation. In some embodiments the lysis solution further comprises such compounds as to ensure that released nucleic acids are compatible with use in downstream assays such as, for example, PCR assays, and in particular DNA genotyping systems.

In the present teachings, dextran magnetic nanoparticles, binding enhancers and polar solvent are then added to the lysate comprising the nucleic acid following the lysis procedure, creating a suspension in which the nucleic acid-polymer-particle complexes are formed, and the nucleic acids are separated and eluted as describe above.

### Binding Solution

A binding enhancer comprising an ionic detergent (for example, N-lauroyl sarcosine (NLS), or lauroyl sarcosinate, also known as sarcosyl, an ionic surfactant derived from sarcosine) and water-soluble long-chain branched polysaccharide (for example dextran 5,000,000 - 40,000,000) can then be added to the suspension. In some embodiments the binding enhancer can comprise one or more of the detergents hexadecyltrimethylammonium bromide or cetyltrimethylammomum bromide (CTAB), deoxycholate, dodecyl β-D-maltoside, nonanoyl-N-methylglucamide, sodium dodecyl sulfate, and polyethylene glycol p-(1,1,3,3-tetramethylbutyl)-phenyl ether (commercially known as Triton® X-100). The binding enhancer may also or alternatively comprise other water-soluble polymers such as, for example, short-chain cellulose, cellulose derivatives, PEG, heparin, starch, glycogen, etc. Alternatively, the magnetic particles can be added to the lysate at the same time as the binding enhancer. In some embodiments, the binding enhancer comprises sarcosyl in the range of 5-15% and dextran in the range of 1-5mg/ml.

A binding solution comprising alcohol, for example ethanol, butanol or isopropanol, can then be added to the suspension. In some embodiments, binding solution comprises 30-100% isopropanol. Alternatively, in some embodiments of the present teachings a single solution comprising binding enhancer and binding solution can be added to the suspension. Binding enhancer, binding solution and cell lysate provide unique conditions such that nucleic acids are entrapped in a non-covalent complex with soluble polymers (such as dextran) having the same or a similar chemical structure as the surface of the magnetic particles. The result is the effective binding of the polymer-entrapped nucleic acids to the magnetic particles in a non-covalent nucleic acid-particle complex. This complex is stable under alcohol wash conditions (such as an ethanol-containing wash solution), and the nucleic acids may easily be later eluted in a standard low salt buffer such as, for example, 10mM Tris buffer, pH 8.0, containing low concentrations of divalent metal ion chelating agents such as EDTA. In some embodiments, this binding stage may be assisted for some types of precipitations by chilling.

### Wash solution

The present teachings relate to a nucleic acid isolation system, such as for genomic DNA, comprising reagents and methods for extraction of the nucleic acids from a biological, food, water, environmental, agricultural, biopharmaceutical or pharmaceutical sample, and comprise a wash step to remove PCR inhibitors from the sample. The wash solution used in the wash step is such as are known in the art. In some embodiments, a particular component of the wash solution can be ethanol in a concentration ranging from 70%-90%. Embodiments of these methods can comprise: formation of a non- covalent complex of nucleic acid (e.g., genomic DNA) with soluble polymers having the same or similar chemical structure as the surface of magnetic particles; binding of the nucleic acid-polymer complex to magnetic particles via interactions between the polymers and surfaces of the particles, thus forming a stable nucleic acid-polymer-particle complex; removal of unbound materials, such as PCR inhibitors, via a wash solution reagent comprising detergent and polar solvent; and elution of the nucleic acid in an aqueous solution amenable to use in downstream applications such as PCR.

Following binding of the entrapped nucleic acids to magnetic particles in the formation of the nucleic acid-particle complexes, a magnetic field can then be applied to the suspension. This magnetic field can be used to remove the nucleic acid-particle complexes from the suspension, forming a complex layer at the bottom or side of the tube and leaving a first supernatant. The first supernatant can then be removed from the tube.

Using a wash solution of detergents and polar solvent to wash the nucleic acid-polymer-particle complex layer that remains after removal of the first supernatant, helps remove any residual salt, nucleotides, chemicals, organic solvents, and other contaminants in the sample, and improves the removal of inhibitors of downstream applications, such as PCR inhibitors. In various embodiments, a wash solution can be used as a wash of nucleic acid-polymer-particle complexes to remove PCR inhibitors and/or contaminants. Solutions useful for washing nucleic acids during isolation and/or purification are well-known to those of skill in the art.

In some embodiments, a wash solution is added to the nucleic acid-polymer-particle complex to create a wash suspension. In some embodiments the wash solution comprises sarcosyl and alcohol (for example, one or more of ethanol, isopropanol, and 70% (v/v) ethanol). In some embodiments the wash solution comprises a detergent such as polysorbate 20 or polysorbate 80. In some embodiments, the wash solution comprises GuSCN in the range of 1-2M; EDTA in the range of 3-5OmM; Tris-HCl in the range of 30-170mM; Antifoam A in the range of 0.001-0.02%; Zwittergent® in the range of 0.3-2%; isopropanol in the range of 30-45%; and sarcosyl in the range of 0.5-2%. The nucleic acid is insoluble in alcohol (such as isopropanol, ethanol and 70% (v/v) ethanol), and remains in a stable complex with the polymers and particles during washing. The washing step can thus be performed vigorously (e.g., by vortex mixing) without risk of loss of the nucleic acids. The sample can then be placed before a magnetic field again, and the resultant wash supernatant comprising contaminants can be removed from the complex layer, which separates out of the wash suspension.

After the washing step, a second wash step can also be performed following similar steps as in the first wash. In some embodiments of the present teachings, following the wash step(s) the nucleic acids are separated and eluted as describe above.

### Nucleic acid extraction and purification

The extraction and purification methods of the present teachings provide useful methods for obtaining nucleic acids such as genomic DNA from biological samples which can be used in downstream applications such as genotyping, quantification, and identification of the source of the biological material, wherein molecular biological processes such as PCR are utilized. The exemplary results described in the Examples herein illustrate the various advantages of the nucleic acid extraction and purification methods of the present teachings, which include but are not limited to providing a nucleic acid (e.g., genomic DNA) preparation that (a) can be derived from a variety of biological materials, (b) is free of detectable inhibitors of downstream applications, such as PCR amplification; (c) can be in concentrated form, and, (d) is amenable for use in any of various applications for nucleic acid analysis, such as genotyping, quantification, detection of the source of biological material, etc. Furthermore, the procedure for extraction and purification of nucleic acids is fully automatable, using standard liquid handling systems.

Additionally, modification of standard alcohol precipitation procedures currently in use, such as those requiring centrifugation, by the methods of the present teachings can provide several clear benefits. First, the methods herein exemplified by the present teachings are faster - the modified procedure for removal of solution from the separated nucleic acid complex takes only about 1-2 minutes, as opposed to about 10-30 minutes for conventional methods using centrifugation. Second, the methods are not reliant upon centrifugation equipment, but can be performed with a simple magnet setup. Third, the methods are more readily suited to automation. For example, a great many tubes could be placed over a large electromagnet and nucleic acids from these could all be isolated simultaneously using, e.g., a multi-channel pipetting device. Fourth, the methods of present teachings are especially effective for step of washing the nucleic acid-polymer-magnetic particle complexes (e.g., in order to remove any residual salt, nucleotides or organic solvents such as phenol), because in the present teachings the washing steps do not require centrifugation and so can be performed rapidly. Additionally, there is minimal or no risk of loss of material, as can occur with the conventional methods based upon centrifugation, where the pellet often detaches from the bottom of the tube during such washing.

### EXAMPLES

Aspects of the present teachings may be further understood in light of the following examples, which should not be construed as limiting the scope of the present teachings in any way.

### Example 1

Samples of human body fluids were obtained in polypropylene tubes of 2.0ml capacity. The samples were 2µl blood, 10µ saliva, and 2µl semen. Each was mixed with a lysis solution in order to lyse cells. The lysis solution comprised 0.0%-1% SDS5 10OmM Tris buffer, and 2 M NaCl, optionally with proteinase K. The lysis mixtures were incubated with or without shaking at a temperature in the range of approximately 60° to 80°C for a period of 40 minutes to 1 hour.

The genomic DNA released from the biological materials was then bound to the magnetic particles having the polyhydroxy groups of dextran. The binding mixture of each sample contained the cell lysate, 10 to 20µl of a suspension comprising the magnetic particles at a concentration of approximately 5-20mg/ml, and 150 to 300µl of a polar compound such isopropanol, ethanol, and/or PEG. The DNA bound to the magnetic particles was then physically separated from the binding mixture by the application of a magnetic field to the mixture.

The DNA, still bound to the magnetic particles in a complex, was then washed with an alcohol-based wash solution (90% Ethanol). The DNA-magnetic particle complexes were again physically separated from the wash mixture by use of a magnetic field. The wash step was repeated one to two times. PCR inhibitors and other macromolecules entrapped in the DNA-magnetic particle complexes were removed in this wash step. The DNA was then released from the magnetic particles by suspending the DNA-particle complexes in 10 to 100µl of aqueous solution, such as DNA-free water or a neutral buffer such as Tris-HCl, and this DNA release mixture was incubated at a temperature in the range of approximately 50 to 75°C. Released genomic DNA was then physically separated from the magnetic particles by use of a magnetic field. The genomic DNA preparation thus obtained was stored at 4°C for short-term storage, or at -20°C for long-term storage. The DNA was quantified by the use of standard methods well-known to those of skill in the art. The results thus obtained, typical for human genomic DNA, are presented in Table 1.

**Table 1:**

| Sample | Yield of DNA, ng |
|---|---|
| 2µl liquid blood | 8 |
| 2µl liquid semen | 250 |
| 10µl liquid saliva | 100 |

### Example 2

Genomic DNA from biological samples was extracted and isolated as described in Example 1, wherein the binding mixture comprised: the cell lysate; 10 to 20µl of the magnetic particle suspension wherein the particles possessed the polyhydroxy groups of dextrans; 10 to 20µl of polyhydroxy polysaccharides such as dextran, cellulose, or soluble starch, at concentrations ranging from approximately 1 to 10mg/ml; 10 to 20µl of anionic detergents, such as N-lauroyl sarcosine, sodium deoxycholate, CTAB, N-dodecyl β -D-maltoside, nonanoyl-N-methylglucamide, Triton® X-100 or sodium dodecyl sulfate; and 150 to 300µl of a polar compound such as isopropanol, ethanol, and/or PEG. The yield of genomic DNA obtained from this method is presented in Table 2.

**Table 2:**

| Sample | Yield of DNA, ng |
|---|---|
| 2µl liquid blood | 140 |
| 2µl liquid semen | 1200 |
| 10µl liquid saliva | 200 |

### Example 3

Genomic DNA from biological samples was extracted and isolated as described in Example 2, wherein the inhibitors and other macromolecules entrapped in the DNA bound to the magnetic particles were removed using two different wash solutions. The step of washing the DNA bound to the magnetic particles comprised one wash with the first wash solution, then one to two wash steps using the second wash solution.

The first wash solution comprised 200 to 500µl of a solution comprising: a mixture of chaotropic salts, such as guanidinium thiocyanate or quanidium hydrochloride, at a concentration ranging from approximately 1 to 2.5M; anionic detergents such as N-lauroyl sarcosine, sodium deoxycholate, CTAB, N-dodecyl β -D-maltoside, nonanoyl-N-methylglucamide, Triton® X-100 and/or sodium dodecyl sulfate; and a polar solvent such as isopropanol or ethanol. The second wash solution comprised ethanol at a concentration in the range of approximately 80 to 95%. The step of washing the DNA bound to the magnetic particles comprised one wash with the first wash solution, then one to two wash steps using the second wash solution.

Wash solution I: 33 mL of BloodPrep™ DNA Purification Solution Applied Biosystems (PN 4342775) + 33 mL of Isopropanol + 1 g sarcosyl + 33 mL deionized water. Wash solution II: BloodPrep™ DNA Wash Solution Applied Biosystems (PN 4342949). As shown in Table 3, it showed that the new wash solution provides
1. a better yield of DNA;
2. the DNA remains bound to the magnetic particles during wash step;
3. the inhibitor of PCR are removed effectively; and
4. it is ease of use since only one wash buffer is required.

**Table 3**

| | 1 Wash with wash solution I and 2 washes with wash solution II. | | 3 Washes with new wash solution (present teaching) | |
|---|---|---|---|---|
| Sample | DNA Yield ng/uL | IPC Ct | DNA Yield ng/uL | **IPC Ct** |
| Blood (2 µL) stained on denim + 2 µL of inhibitor mix that contained indigo (12.5 mM), hematin (0.5 mM), humic acid (2.5 mg/mL) and urban dust extract. | 0.67 | 29.35 | 0.71 | **29.23** |

### Example 4

Samples of the human body fluids 2µl of blood, 20µl of saliva, and 1 µl of semen were obtained in a polypropylene tube of 2.0ml capacity and mixed with a lysis solution. The lysis mixture was incubated with or without shaking at a temperature in the range of approximately 60 to 80°C for a period of approximately 40 minutes to 4 hours.

The genomic DNA released from the biological materials was then bound to the magnetic particles comprising the polyhydroxy groups of dextran. The binding mixture comprised: the cell lysate; 10 to 20µl of the suspension comprising the magnetic particles at a concentration in the range of approximately 5-20mg/ml; 10 to 20µl of polyhydroxy polysaccharides, such as dextran, cellulose, and/or soluble starch at a concentration in the range of approximately 1 to 10mg/ml; approximately 10 to 20µl of anionic detergents, such as N-lauroyl sarcosine, sodium deoxycholate, CTAB, N-dodecyl β-D-maltoside, nonanoyl-N-methylglucamide, Triton® X-100, and/or sodium dodecyl sulfate; and approximately 150 to 300µl of a polar compound such as isopropanol, ethanol, and/or PEG.

The DNA bound to the magnetic particles was physically separated from the binding mixture by the use of a magnetic field. The DNA bound to the magnetic particles was then washed with a wash solution comprising detergent such as sodium deoxycholate, N-lauroyl sarcosine, polysorbate 20 or polysorbate 80 (commercially known at Tween® 20 or Tween® 80, respectively), or Triton® X-100 at a concentration in the range of approximately 0.05 to 1%, in a polar solvents such as ethanol or isopropanol at a concentration in the range of approximately 65 to 80%.The DNA bound to the magnetic particles was physically separated from the wash mixture by use of a magnetic field. The wash step was repeated one to two times. The PCR inhibitors and other macromolecules entrapped in the DNA bound to the magnetic particles were removed in this step.

The DNA was then released from the magnetic particles by suspending the DNA bound to the magnetic particles in approximately 10 to 100µl of aqueous solution, such as DNA-free water; or buffer comprising Tris-HCl at a molarity in the range of approximately 10 to 50mM and pH in the range of approximately 7.0 to 8.5 and the chelating agent EDTA at a concentration in the range of approximately 0.1 to 3.0mM. The release mixture was incubated at a temperature in the range of approximately 50 to 75°C. Released genomic DNA was then physically separated from the magnetic particles by use of a magnetic field. The genomic DNA preparation thus obtained was stored at 4 °C for short-term storage, or at -20°C for long-term storage. The DNA was quantified using standard methods well-known to those of skill in the art such as, for example, quantification of human DNA using the Quantifiler® Human DNA Quantification Kit. Results thus obtained, typical for human genomic DNA, are presented in Table 4.

**Table 4:**

| Sample | Yield of DNA, ng |
|---|---|
| 2µl liquid blood | 145 |
| 1µl liquid semen | 650 |
| 20µl liquid saliva | 165 |

### Example 5

Genomic DNA was isolated as described in Example 4, wherein the biological fluids were 2µl of blood, 20µl of saliva and 1µl of semen, and the fluids were stained on fabric such as cotton cloth, polyester cloth or denim. Results thus obtained for fluids stained on cotton, typical for human genomic DNA, are presented in Table 5.

**Table 5:**

| Sample | Yield of DNA, ng |
|---|---|
| 2µl blood on cotton | 80 |
| 1µl semen on cotton | 550 |
| 20µl saliva on cotton | 145 |

### Example 6

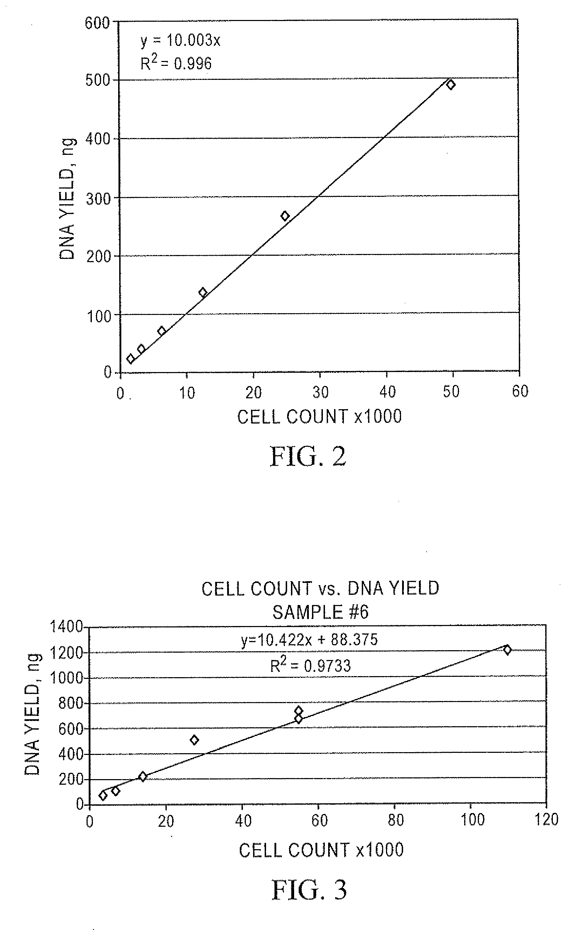

Genomic DNA was isolated from cultured Raji cells at cell counts ranging from 1562 to 50000 cells, as described in Example 4. Results thus obtained, typical for human genomic DNA, are presented in Fig. 2 and Table 6.

**Table 6:**

| Raji Cells, cell count | DNA yield, ng |
|---|---|
| 50000 | 488 |
| 25000 | 266 |
| 12500 | 136 |
| 6250 | 69.7 |
| 3125 | 37.3 |
| 1562 | 21 |

### Example 7

Genomic DNA was isolated from cultured K562 cells at cell counts ranging from approximately 3500 to 110000 cells, as described in Example 4. Results thus obtained, typical for human genomic DNA, are presented in Fig. 3 and Table 7.

**Table 7:**

| K562 Cells, cell count | DNA yield, ng |
|---|---|
| 110000 | 1190 |
| 55000 | 700 |
| 27500 | 500 |
| 1.3750 | 217 |
| 6875 | 114 |
| 3438 | 65 |

### Example 8

Genomic DNA was isolated from biological fluids and stains of biological fluids on different substrates, such as cloth, FTA paper, a swab and denim, as described in Example 4, wherein the lysis of biological material was performed by suspending the biological material in a lysis solution. The lysis solution was incubated at a temperature in the range of 50 to 60°C for a period of 40 minutes to 2 hours, and DNA was obtained and quantified by methods as outlined in Example 4.

### Example 9

Genomic DNA was isolated as described in Example 4, wherein approximately 2 to 10µl of the biological fluid blood was spotted on cotton, and was enriched with 1 to 5µl of PCR inhibitors comprising hematin to a final concentration ranging from approximately 0.1 to 2mM, humic acid to a final concentration ranging from approximately to 5mg/ml, indigo to a final concentration ranging from 5 to 20mM, and urban dust extract to a final concentration ranging from approximately 2 to 12mg/ml. The PCR inhibitors were effectively removed during the extraction and isolation of the genomic DNA by these methods, as evidenced by measuring the Cₜ value of the internal PCR control (IPC) using the Quantifiler® Human DNA Quantification Kit. Results thus obtained are presented in Table 8.

**Table 8:**

| Sample | IPC Cₜ |
|---|---|
| 2µl blood dried on cotton | 27.58 |
| 2µl blood dried on cotton in presence of inhibitors mix | 27.60 |
| Extraction Blank | 27.77 |

### Example 10

Genomic DNA was isolated as described in the Example 4, wherein the biological samples comprising stains of biological fluids underwent environmental insults by exposure to the environment for a period of 1 to 7 days. DNA was isolated and quantified as per the methods of Example 4.

### Example 11

Genomic DNA was isolated as described in Example 4, wherein the biological samples were of varying nature, comprising buccal swabs on the materials cotton, rayon, and nylon; blood, saliva and semen stains on materials like cotton cloth, denim, polyester cloth, FTA paper, filter paper; cigarette buts, swab of finger prints; mixtures of body fluids like epithelial cells and semen; swabs of body fluids on different surfaces.

### Example 12

Genomic DNA was isolated from biological samples, as described in Example 11, and were processed for quantification of human DNA using the Quantifiler® Human DNA Quantification Kit. The results for the quantity of human DNA and the Cₜ of the IPC, which measures the presence of PCR inhibitors from some typical substrates containing biological materials, are presented in Table 8. A positive difference greater than 1 in the IPC Cₜ value for a sample DNA preparation relative to the IPC Cₜ value of the negative template control (NTC) indicates the presence of PCR inhibitors.

**Table 9:**

| Sample | DNA Yield, ng | IPC Cₜ |
|---|---|---|
| Blood 2µl | 62 ±13 | 27.6 |
| Blood Stain 2µl on denim | 76 ±15 | 27.9 |
| Blood Stain 2µl on rayon | 39.5 ±8 | 27.7 |
| Blood Stain 2µl on nylon | 39 ±10 | 27.6 |
| Saliva 5 µl | 158 ±30 | 27.6 |
| Saliva stain 5 µl on cotton | 143 ±25 | 27.7 |
| Semen 2µl | 1250 ±150 | 27.6 |
| Semen stain 2µl on cotton | 1340 ±130 | 27.7 |
| Cigarette butt | 154 ±40 | 27.8 |
| Chewing Gum | 21 ±8 | 27.9 |
| Extraction Blank | 0 | 27.6 |
| NTC | | 27.6 |

### Example 13

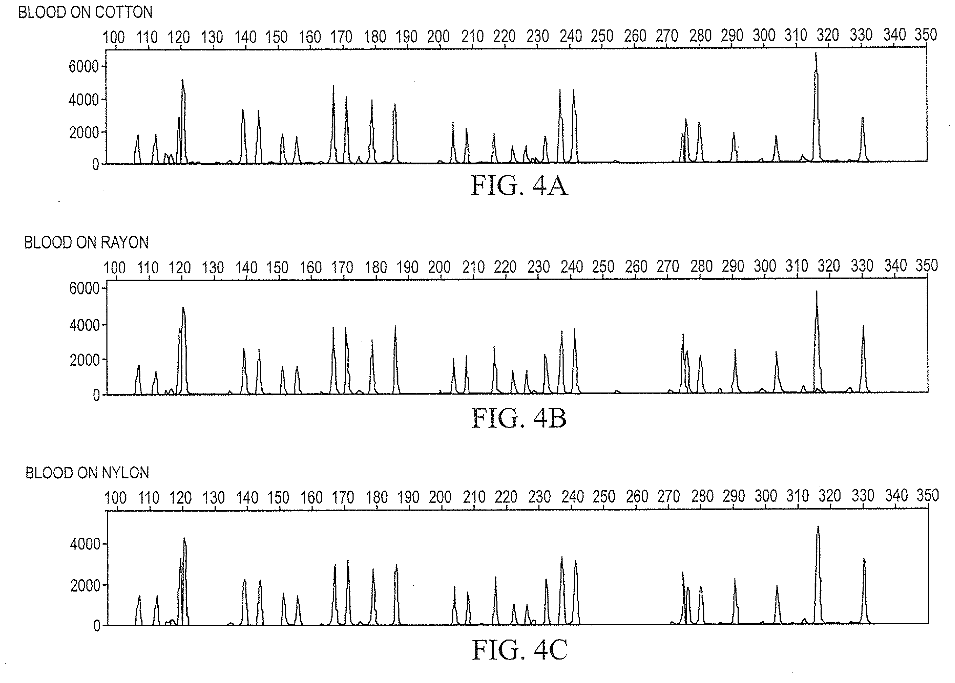

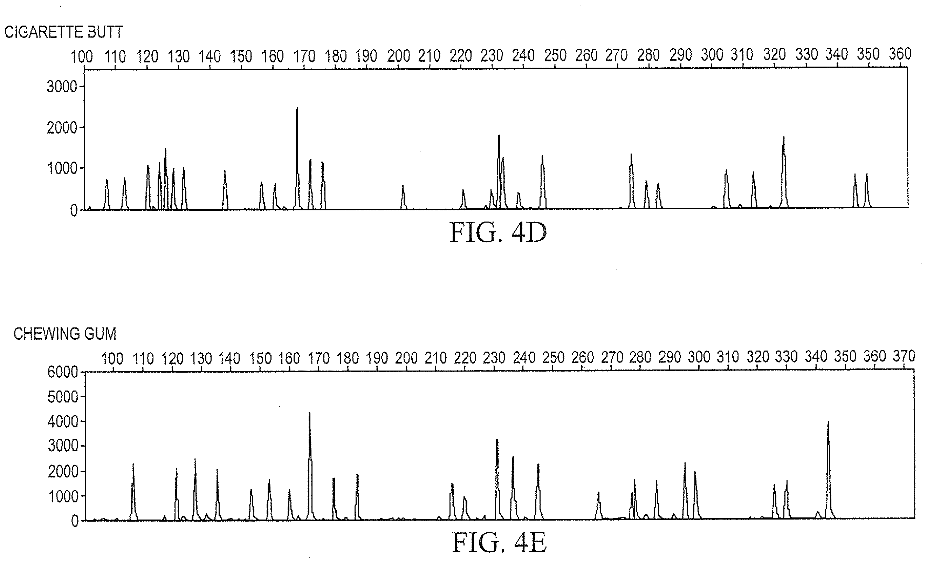

Genomic DNA was isolated from biological samples as described in Example 11, and were processed for genotyping using an AmpF/STR® kit, such as Identifiler®. The genotype profiles thus obtained from some typical substrates containing biological materials are presented in Fig. 4.

### Example 14

Genomic DNA was isolated from biological samples as described in Examples 1 to 12, and were processed using the reagents as described therein in the form of a kit.

### Example 15

Isolating spiked DNA from ground beef or hamburger was enhanced with an enrichment step.

### Chemical lysis Protocol:

The sample was placed in a 1.5 mL microcentrifuge tube, and 300ul of lysis Solution was added and the mixture was vortexed for 15 seconds until the sample was completely resuspended, and then incubated at 70°C for 20 minutes. Next, the tube was vortexed at maximum speed for 10 seconds and the magnetic particles were vortexed until they were in a suspension, about 5 minutes. 30ul of particle suspension was added, the sample/particle mixture was vortexed at low speed, 10 sec. and then add 190ul of binding solution added and vortexed for 5 seconds followed by a 7 minute incubation at room temperature with continuous shaking. The mix was then vortexed at low speed for 10 seconds and the tube placed in a magnetic separator, separation occurred in 1-2 minutes. With care the liquid phase was removed and discarded without disturbing the magnetic particles pellet. 300ul of wash solution was added followed by vortexing at medium speed for 5 sec or until the pellet was resuspended and then the tube was replaced in the magnetic separator for 30 seconds and the liquid phase was again carefully discarded without disturbing the magnetic particle pellet. The step was repeated twice more. 50ul elution buffer is added and the tube was vortexed at medium speed 5 seconds and then incubated at 70°C for 5 minutes, followed by a second vortex and then at least 1 minute in the magnetic separator. The supernatant was transferred to a new tube.

### Proteinase K lysis Protocol (recommended when processing of difficult-to- dissolve, proteinrich, tissue samples, Gram+ bacteria containing samples is required):

Place sample in to a 1.5 mL microcentrifuge tube, to the tube was added 200 ul of PK buffer and 10 ul of proteinase K (20mg/ml), followed by incubation at 56°C for 20 minutes. 400ul of lysis solution was added followed by vortexing at medium for 15 seconds until the sample is resuspended and then vortexed at maximum speed for 10 seconds. The stock magnetic particles were prepared as before and then 30ul were added to the sample with vortexing at low speed 10 seconds. 400ul of binding solution was added and again vortexed for 5 seconds followed by continuous shaking at room temperature for 10 minutes. The sample was then vortexed again at low speed for 10 seconds and placed in the magnetic separator for 1-2 minutes until separation was complete. Removal and washing were performed as described for a total of 3 washes. The magnetic particles were allowed to air-dry for 5 minutes, lid opened. 50ul elution buffer was added, lid closed and vortexed at medium for 5 seconds followed by incubation at 70°C for 5 minutes. The elution volume can be increased up to 200ul, if necessary. The tube was then vortexed at medium speed for 2 seconds and placed in the magnetic separator for 1 minute. The DNA was in the elution fluid and used in the PCR reaction vs. a competitor kit. The result was in three of three samples the competitor Ct was almost 2 Ct greater than the disclosed method. Thus, the disclosed method has greater sensitivity and resulted in great DNA recovery than a competitor.

### Example 16

Mycoplasma isolation was performed by placing 2 to 10 ml of sample into a 50 ml tube followed by centrifuging at 1000 x g for 5 min. The supernatant was transferred to a 50 ml tube and kept on ice.

To the pellet was resuspended gently with a Pasteur pipet in 5 ml of ice-cold 1X PBS and then centrifuged at 1000 x g for 5 min., the supernatant was carefully removed and discarded. The pellet was gently resuspended in 550 ul ice-cold binding buffer taking care to avoid cell lysis. The suspension was transferred to a 2 ml tube and incubated on ice for about 5 min. followed by centrifuging at 1000 x g for 5 min. at 4°C. 400ul of the supernatant was transferred to a 2 ml tube followed by centrifuging at 1000 x g for 3 min. at 4°C. With a P200 pipet tip, 300ul of supernatant was transferred to a 2ml tube and stored on ice.

The supernatant in the 50 mil tube on ice was at 4°C centrifuged at 16,000 x g for 30 min. at 4°C. The supernatant was aspirated and discarded taking care to not disturb the pellet. The cell fraction lysate from the 2 ml tube was transferred to the pellet in the 50 ml tube and the pellet was gently resuspended. The suspended pellet was transferred to a 2 ml tube to which 0.5 M EDTA and 18 ul of RNase Cocktail was added (PrepSEQ™ Mycoplasma Nucleic Acid Extraction Kit, Applied Biosystems, Foster City, CA), vortexed briefly and then microfuged briefly. The tube was then incubated at 56°C for 30 minutes with vortexing twice during the incubation. 5ul of proteinase K was added followed by a brief vortex and spin with another incubated at 56°C for 10 minutes. 700ul of lysis buffer was added followed by vortexing at medium speed for 5 seconds. The resulting 1 ml of lysate was used for DNA extraction as described in Example 15.

The extracted Mycoplasma DNA was evaluated in by PCR and melt-curve analysis. The results indicated that the wash solution did not significantly impact the PCR or the recovery of the DNA and the extraction and isolation methods disclosed resulted in a better DNA yield than the competing kit with better sensitivity as the competing kit failed to detect the Mycoplasma DNA while all tested samples with the claimed invention detected Mycoplasma DNA.

## Claims

1. A method of making a product, wherein the product comprises a nucleic acid, comprising the steps of:
(a) lysing cells from a sample in a lysis solution, thus forming a lysate comprising the nucleic acid;
(b) treating the lysate with a starting solution comprising a water-soluble polyhydroxy polymer and a detergent;
(c) applying suspended magnetically attractable dextran-encased magnetite nanoparticles to form a nucleic acid-polymer-particle complex;
(d) washing the complex with a wash solution while applying a magnetic field; and
(e) eluting and recovering the nucleic acid,
wherein steps (b) and (c) may be carried out simultaneously.

2. The method of claim 1, wherein the nucleic acid is derived from a sample of biological material comprising one or more of blood, blood stain, saliva, saliva stain, buccal cells, buccal swab, semen, semen stain, cigarette butt, chewing gum, ground beef, brie cheese, raw chicken, shrimp, cantaloupe, dry infant formula, whole shell eggs, ground black pepper, dry pet food, peanut butter, orange juice, pasteurized milk, alfalfa sprouts, roast beef, smoked salmon, mayonnaise, salad dressing, milk, ice cream, cured bacon, lettuce, sausages, beet trim, juices, spinach, cheddar cheese, raw milk, oysters, clams, mussels.

3. A kit for isolating and purifying nucleic acid from a sample comprising biological material, comprising starting solution comprising a water-soluble polyhydroxy polymer and a detergent; and dextran-encased magnetite nanoparticles.

## Patentansprüche

1. Verfahren zur Herstellung eines Produkts, wobei das Produkt eine Nukleinsäure umfasst, umfassend die Schritte:
(a) Lysieren von Zellen aus einer Probe in einer Lyselösung, wodurch ein Lysat gebildet wird, das die Nukleinsäure umfasst;
(b) Behandeln des Lysats mit einer Ausgangslösung, die ein wasserlösliches Polyhydroxypolymer und ein Detergens umfasst;
(c) Anwenden von suspendierten, magnetisch anziehbaren, Dextran-umhüllten Magnetit-Nanopartikeln, um einen Nukleinsäure-Polymer-Partikelkomplex zu bilden;
(d) Waschen des Komplexes mit einer Waschlösung während ein Magnetfeld angelegt wird; und
(e) Eluieren und Gewinnen der Nukleinsäure,
wobei die Schritte (b) und (c) gleichzeitig ausgeführt werden können.

2. Verfahren nach Anspruch 1, wobei die Nukleinsäure aus einer Probe eines biologischen Materials abgeleitet ist, die eines oder mehrere von Blut, Blutfleck, Speichel, Speichelfleck, bukkale Zellen, bukkalem Abstrich, Samen, Samenfleck, Zigarettenstummel, Kaugummi, Rinderhack, Brie-Käse, rohem Huhn, Garnelen, Zuckermelone, trockener Säuglingsnahrung, ganze Schaleneier, gemahlenem schwarzen Pfeffer, Trockentierfutter, Erdnussbutter, Orangensaft, pasteurisierter Milch, Alfalfasprossen, Roastbeef, Räucherlachs, Mayonnaise, Salatdressing, Milch, Speiseeis, geräuchertem Speck, Kopfsalat, Würsten, Rübenschnitt, Säften, Spinat, Cheddar-Käse, Rohmilch, Austern, Muscheln, Miesmuscheln umfasst.

3. Kit zum Isolieren und Reinigen von Nukleinsäure aus einer Probe, die biologisches Material umfasst, umfassend eine Ausgangslösung, die ein wasserlösliches Polyhydroxypolymer und ein Detergens umfasst; und Dextran-umhüllte Magnetit-Nanopartikel.

## Revendications

1. Procédé de préparation d'un produit, dans lequel le produit comprend un acide nucléique, comprenant les étapes consistant à :
(a) lyser les cellules d'un échantillon dans une solution de lyse, formant ainsi un lysat comprenant l'acide nucléique ;
(b) traiter le lysat avec une solution de départ comprenant un polymère polyhydroxylé hydrosoluble et un détergent ;
(c) appliquer des nanoparticules de magnétite encapsulées dans du dextrane magnétiquement attirables et en suspension pour former un complexe d'acide nucléique-polymère-particules ;
(d) laver le complexe avec une solution de lavage tout en appliquant un champ magnétique ; et
(e) éluer et récupérer l'acide nucléique,
les étapes (b) et (c) pouvant être réalisées de manière simultanée.

2. Procédé selon la revendication 1, dans lequel l'acide nucléique est dérivé d'un échantillon de matériel biologique comprenant un ou plusieurs éléments choisis parmi du sang, une tache de sang, de la salive, une tache de salive, des cellules buccales, un prélèvement buccal, du sperme, une tache de sperme, un mégot de cigarette, un chewing-gum, du boeuf haché, du brie, du poulet cru, des crevettes, du melon cantaloup, du lait infantile en poudre, des oeufs entiers avec coquille, du poivre noir moulu, des aliments déshydratés pour animaux, du beurre de cacahuète, du jus d'orange, du lait pasteurisé, des germes de luzerne, du rôti de boeuf, du saumon fumé, de la mayonnaise, de la vinaigrette, du lait, des glaces, du bacon séché, de la laitue, des saucisses, de la betterave râpée, des jus, de l'épinard, du cheddar, du lait cru, des huîtres, des palourdes, des moules.

3. Kit pour isoler et purifier un acide nucléique à partir d'un échantillon comprenant du matériel biologique, comprenant une solution de départ comprenant un polymère polyhydroxylé hydrosoluble et un détergent ; et des nanoparticules de magnétite encapsulées dans du dextrane.
